# EUROPÄISCHE PATENTANMELDUNG

(11) **EP 2 926 864 A1**
(43) Veröffentlichungstag der Anmeldung: **07.10.2015**
(21) Anmeldenummer: 15159326.6
(22) Anmeldetag: 17.03.2015
(51) Int. Cl.: A61N 1/375, A61N 1/37, H01G 4/35, H01M 2/06

(54) **Elektrische Durchführung für elektromedizinische Implantate, elektrisches Kontaktelement mit einer solchen Durchführung sowie elektromedizinisches Implantat**

(30) Priorität: 02.04.2014 US 201461973878 P
(71) Anmelder: BIOTRONIK SE & Co. KG, 12359 Berlin (DE)
(72) Erfinder: Kronmüller, Daniel, 90409 Nürnberg (DE); Teske, Josef, 96103 Hallstadt (DE)
(74) Vertreter: Galander, Marcus

(57) **Zusammenfassung**

Die Erfindung betrifft eine elektrische Durchführung (100) für elektromedizinische Implantate, umfassend mindestens einen elektrischen Kontakt (40) zum Verbinden mit einem Gegenkontakt. Der mindestens eine elektrische Kontakt (40) ist als Leiterbahn (42) ausgebildet, die sich zumindest bereichsweise in oder auf einem dielektrischen Substrat (10) von einem ersten Bereich (20) zu einem zweiten Bereich (22) erstreckt, wobei das Substrat (10) beim Übergang vom ersten in den zweiten Bereich (20,22) durch einen Flansch (70) geführt ist, und dass das Substrat (10) mit dem Flansch (70) hermetisch dicht verbunden ist.

Die Erfindung betrifft ferner ein elektrisches Kontaktelement sowie ein elektromedizinisches Implantat mit einer solchen Durchführung.

## Beschreibung

Die Erfindung betrifft eine elektrische Durchführung für elektromedizinische Implantate, ein elektrisches Kontaktelement mit einer solchen Durchführung sowie ein elektromedizinisches Implantat mit einem derartigen Kontaktelement.

Medizinische Implantate sind in großer Vielfalt aus dem Stand der Technik bekannt. Als elektromedizinisches Implantat wird im Zusammenhang mit der vorliegenden Erfindung ein Implantat verstanden, das neben einer Stromversorgung (z.B. Batterie) weitere elektrische und/oder elektronische Bauteile umfasst, welche in einem Gehäuse angeordnet sind, das hermetisch dicht abgeschlossen ist.

Da jeder Eingriff für den Patienten eine Belastung bedeutet, werden an Implantate erhöhte Anforderungen an Langlebigkeit und Sicherheit gestellt. Auf der Außenseite des Implantats dürfen nur Materialien eingesetzt werden, welche biokompatibel sind, das heißt, welche nicht durch den menschlichen Organismus aufgenommen oder verstoffwechselt werden können. Bekannte biokompatible Materialien sind z.B. Titan oder Platin.

Derartige elektromedizinische Implantate sind beispielsweise Herzschrittmacher, Defibrillatoren, Neurostimulatoren, Herzschrittmacher, Kardioverter/Defibrillatoren und Cochlea-Implantate.

Nach der Implantation in einen Patienten therapieren die Implantate, indem sie die elektronischen Impulse des Körpers überwachen bzw. aktiv stimulieren. So werden beispielsweise Stimulationsimpulse oder Defibrillatorschocks an bestimmte Körperstellen übertragen oder abgeben. Ferner können die elektronischen Potentiale von Körperstellen erfasst und aufgezeichnet werden und nach elektronischer Auswertung über eine Antenne an ein Überwachungssystem, z.B. ein so genanntes Home Monitoring System, dem behandelten Arzt zur Verfügung gestellt werden. Für all diese Zwecke sind elektrische Verbindungen von der Außenseite des Implantates zu dem hermetisch versiegelten Innenbereich notwendig, in welchem die Signalverarbeitung stattfindet.

Aus dem Stand der Technik, z.B. der Druckschrift EP 1 897 588 B1 oder der US 2013/0309237 A1, sind Durchführungen für elektromedizinische Implantate bekannt, bei denen zur elektrischen Signalführung metallische Stifte durch einen Keramikkörper durchgeführt sind. Diese müssen aufwendig eingesetzt und verlötet werden. Die metallischen Lote bilden Menisken aus, so dass aufgrund der notwendigen Isolationsstrecken die Pins nicht sehr dicht gepackt sein können. Weiterhin sind die eingesetzten Stifte in ihrer Position zueinander und relativ zu der Keramik bzw. dem Gehäuse nicht stabil, da die Stifte sich leicht deformieren. Dies erschwert die maschinelle und automatisierte Anbindung und verursacht zusätzliche Kosten. Ferner ist nachteilig, dass Platine und Keramikdurchführungen jeweils in separaten Fertigungsschritten hergestellt werden müssen und aufwendig gefügt und die elektrischen Verbindungen verknüpft werden müssen. Bei dem Einsatz von EMI-Filtern mit einer solchen Durchführung werden aufwändige, Kosten und Fehler verursachende Fügeprozesse (Weichlöten, Kleben, Schweißen) eingesetzt, um EMI-Filter an Durchführungen anzubringen. Hierfür sind u. A. auch hinsichtlich des Platzes aufwändige Zusatzbauteile (Kondensatoren) an den Anschlussbändern notwendig.

Gleiches gilt auch für die ebenfalls bekannten Glasdurchführungen oder Glas-Keramikdurchführungen, wobei bei manchen Materialkombinationen der Glasdurchführungen zusätzlich die Kontrolle des Glaslotflusses schwierig ist. Bei beiden Durchführungsarten sind Einschränkungen hinsichtlich der machbaren Geometrie und der Wahl der Werkstoffe aufgrund der im Allgemeinen hohen Löttemperatur und der beteiligten Wärmeausdehnungskoeffizienten notwendig.

Der Erfindung liegt die Aufgabe zugrunde, eine hochwertige und robuste elektrische Durchführung für elektromedizinische Implantate zu schaffen, die reproduzierbar herstellbar ist.

Eine weitere Aufgabe der Erfindung ist das Bereitstellen eines elektrischen Kontaktelements mit einer solchen Durchführung.

Eine weitere Aufgabe ist das Bereitstellen eines elektromedizinischen Implantats mit einem solchen Kontaktelement.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine elektrische Durchführung für elektromedizinische Implantate vorgeschlagen, umfassend mindestens einen elektrischen Kontakt zum Verbinden mit einem Gegenkontakt, der mindestens eine elektrische Kontakt als Leiterbahn ausgebildet ist, die sich zumindest bereichsweise in oder auf einem dielektrischem Substrat von einem ersten Bereich zu einem zweiten Bereich erstreckt, wobei das Substrat beim Übergang vom ersten in den zweiten Bereich durch einen Flansch geführt ist, und dass das Substrat mit dem Flansch hermetisch dicht verbunden ist.

Insbesondere trennt die Dichtung die beiden Bereiche hermetisch dicht voneinander.

Die Produktion einer solchen Durchführung ist kostengünstiger als konventionelle Mehrpinvarianten, bei denen die elektrischen Kontakte durch einzelne massive Drahtpins gebildet sind. Im Gegensatz dazu weist die Erfindung elektrische Kontakte auf, die als Leiterbahnen auf dem starren Substrat in ihrer Lage fixiert sind und z.B. sich nicht gegeneinander verbiegen oder verbogen werden können. Das Substrat kann einlagig sein, oder es können zwei oder mehr Lagen aufeinander angeordnet sein, wobei jede Lage Leiterbahnen aufweisen kann. Vorteilhaft sind Durchkontaktierungen zwischen verschiedenen Substratlagen möglich.

Im Betrieb sind sie weniger fehleranfällig als Mehrpinvarianten. Die mögliche Kontaktdichte ist deutlich höher als bei konventionellen Durchführungen. Es kann eine Datenrate erreicht werden, die bei der erfindungsgemäßen Durchführung deutlich höher sein kann, als bei Drahtpins ist. Die relative Ausrichtung aller elektrischen Kontakte zueinander ist gewährleistet. Impedanzeigenschaften der Durchführung sind gezielt einstellbar und können verbessert sein. Ebenso kann eine elektromagnetische Schirmung besser gewährleistet sein. Die elektrischen Eigenschaften wie Widerstand, Schirmung und Impedanz sind mit Hilfe des Substrates bereits während des Designs planbar und optimierbar.

Durch die hermetische Dichtigkeit der Verbindung zwischen Substrat und Flansch können die hohen Anforderungen an medizinische Implantate erfüllt werden. Insbesondere ist die Durchführung virendicht, wasserdampfdicht und gasdicht. Eine solche hermetisch dichte Durchführung erfüllt auch die Anforderungen für vakuumdichte Durchführungen, insbesondere für ultrahochvakuumdichte Durchführungen.

Insbesondere kann das Substrat eine bedruckte, einlagige oder mehrlagige Platine sein. Die Verwendung von technischen Keramiken als Substratmaterial ist besonders günstig. Dabei können so genannte LTCC-Keramiken (LTCC = Niedertemperatur-Einbrand-Keramik) oder HTCC-Keramiken (HTCC = Hochtemperatur-Einbrand-Keramik) eingesetzt werden. Es können Leiterbahnen und/oder Schaltkreise auf mehreren Ebenen aus keramischen Substratschichten im Stadium eines Grünlings sowie eine Metallisierung zusammengefügt und anschließend gemeinsam gesintert werden. Als Basismaterial stehen verschiedene technische Keramiken (z.B. Glas-Keramik, AIN, Al₂O₃) zur Verfügung, die zuerst strukturiert werden können. Anschließend werden sie mit hochschmelzenden Materialien (z.B. Au, Pd, Pt, Ir, Nb, Ta, W, Mo oder Legierungen aus zwei oder mehr Bestandteilen daraus) beschichtet bzw. bedruckt, bevor sie zu zwei oder mehreren Lagen gestapelt und gesintert werden. Typische Dicken von Einzellagen liegen zwischen 100 µm bis 200 µm, insbesondere zwischen 100 µm und 150 µm.

Vorteilhaft wird eine Durchführung mit mehreren elektrischen Kontaktelementen erreicht, bei der sich die Kontaktelemente, anders als übliche Drahtpins, nicht verbiegen und nicht gerichtet werden müssen. Zusätzliche Kosten, die durch das Ansetzen eines Steckers an eine Platine entstehen, können vermieden werden. Diese entstehen, weil z.B. eine neue Isolationskeramik eingesetzt werden muss, und zusätzlich Pins bzw. Stifte durch die Keramik geführt werden müssen, um eine definierte elektrische Verbindung herzustellen. Für eine solche bekannte Durchführung sind technologisch viele Einzelprozesse erforderlich. Vorteilhaft erlaubt die erfindungsgemäße Durchführung ferner, dass die Anordnung der Leiterbahnen und der elektrischen Kontakte im ersten Bereich von derjenigen der Leiterbahnen im zweiten Bereich abweicht.

Die bekannte fertige Durchführung ist zudem anfällig für Deformation der Pins, was kostenintensive Nacharbeitsmaßnahmen notwendig macht. Die elektrischen Eigenschaften der Pins bzw. Stifte zueinander sind von der Position der Stifte abhängig und können leicht und in unerwünschter Weise variieren. Ein Übergang von Platine zur Durchführung verändert die elektrischen Eigenschaften sowie die Schirmung ebenfalls und kann Verursacher für EMV-Probleme sein, da die ungeschirmten Pins als Antennen fungieren. Vorteilhaft vermeidet oder vermindert die erfindungsgemäße Durchführung derartige Probleme.

Das Substrat dient als Träger für Schaltungen, positioniert die Leiterbahnen, welche die Pins ersetzen, relativ und absolut im Stecker. Ferner kann das Substrat als federndes Element dienen, welches durch Schrägstellung einen leichten Anpressdruck auf den Gegenstecker erzeugt. Die elektrischen Eigenschaften im Substratmaterial können mit hoher Genauigkeit und reproduzierbar eingestellt werden und unterliegen keiner relevanten Änderung durch Alterung oder Verschleiß. Durch die Fertigung der Durchführung mit dem Substratmaterial können EMV-Probleme verringert werden. Hohe Daten- bzw. Übertragungsraten werden durch entsprechende Anordnung der Kontakte und Leiterbahnen ermöglicht.

Es ist eine deutlich höhere Packungsdichte der Durchführung möglich. Teure Arbeitsprozesse während der Produktion, wie Ausrichten von Pins, Pinbiegen, entfallen. Die Durchführung kann gegenüber EMV-Störungen besser geschirmt werden.

Gemäß einer günstigen Ausgestaltung kann zur hermetisch dichten Verbindung ein Lot, insbesondere ein Glaslot oder Metalllot, vorgesehen sein. Hierzu können vorteilhaft biokompatible Materialien eingesetzt werden, z.B. Goldlot, biokompatibles Glas, eine Legierung aus TiCuNi.

Gemäß einer günstigen Ausgestaltung kann das Substrat eine Leiterplatte, insbesondere eine keramische Leiterplatte, insbesondere eine Leiterplatte aus LTCC- oder HTCC-Keramik, sein. Vorteilhaft können Schaltungsanordnungen und Bauelementanordnungen eingesetzt werden, wie sie in der Elektronikherstellung bekannt und bewährt sind.

Gemäß einer günstigen Ausgestaltung kann das Substrat ein oder mehrere gedruckte Schaltungen aufweisen. Vorteilhaft lassen sich auf diese Weise verschiedene Funktionen in die Durchführung integrieren, beispielsweise Hochfrequenzfilter, Kapazitäten und dergleichen. Ebenso kann das Substrat gemäß einer günstigen Ausgestaltung ein oder mehrere SMD-Bauelemente aufweisen, welche auf eine Substratoberfläche montiert sind. Es lassen sich vorteilhaft günstige Schaltungen aus integrierten und/oder diskreten elektrischen Komponenten darstellen.

Gemäß einer günstigen Ausgestaltung kann wenigstens der erste Bereich des Substrats eine elektromagnetische Schirmung aufweisen. Dies kann durch eine vollständige metallische oder metallisierte Umrandung des ersten Bereichs, ähnlich wie bei einem USB2.0 oder HDMI-Stecker erfolgen, oder es kann eine Schirmung innerhalb des Substrats vorgesehen sein. Es kann auch vorgesehen sein, dass nur der zweite Bereich eine Schirmung aufweist.

Gemäß einer günstigen Ausgestaltung kann das Substrat ein oder mehrere elektrische Kontakte auf einer oder mehreren Oberflächen aufweisen. So können elektrische Kontakte auf den Hauptflächen, aber auch auf Seitenflächen oder auch Stirnseiten vorgesehen sein. Gemäß einer weiteren günstigen Ausgestaltung kann das Substrat im ersten und/oder zweiten Bereich eine gestufte Oberfläche aufweisen mit wenigstens zwei Stufen. Dies erlaubt vorteilhaft eine Erweiterung der möglichen elektrischen Kontaktmöglichkeiten. Ebenso wird eine eindeutige Einbaulage der Durchführung erreicht. Dies ist insbesondere vorteilhaft, wenn Leiterbahnen und/oder Schaltkreise innerhalb des Substrats vorgesehen sind, so dass es notwendig ist, den ersten Bereich vom zweiten Bereich des Substrats zu unterscheiden.

Gemäß einer günstigen Ausgestaltung kann das Substrat im ersten und/oder zweiten Bereich wenigstens eine Ausnehmung so aufweisen, dass zwei oder mehr voneinander beabstandete Substratsegmente gebildet sind. Diese Variante erlaubt ebenso eine Erweiterung der möglichen elektrischen Kontaktmöglichkeiten und eine eindeutige, verwechslungssichere Einbaulage und Anbindung der Durchführung.

Gemäß einer günstigen Ausgestaltung kann das Substrat und/oder können die elektrischen Kontakte zumindest bereichsweise aus biokompatiblem Material gebildet sein oder zumindest bereichsweise durch biokompatibles Material gekapselt sein. Vorteilhaft kann das Substrat aus Aluminiumoxid Al₂O₃, Siliziumdioxid SiO₂, Aluminiumnitrid AIN, einer LTCC-, HTCC-Keramik oder einer Glas-Keramik gebildet sein oder wenigstens bereichsweise damit beschichtet sein.

Gemäß einer günstigen Ausgestaltung kann eine Zuordnung der elektrischen Kontakte in den beiden Bereichen unterschiedlich bzw. vertauscht ausgeführt ist, indem die Leiterbahnen im Substrat in verschiedenen Leiterbahn-Ebenen mittels Durchkontaktierungen elektrisch isoliert und gekreuzt ausgeführt werden. Dies erlaubt ein flexibles Design bei der Kontaktierung der Vorrichtung.

Gemäß einer günstigen Ausgestaltung kann das Metall der elektrischen Leiterbahn ein Metall aus der Gruppe Gold, Platin, Iridium, Palladium, Niob, Tantal, Wolfram, Titan, Kupfer, Nickel oder eine Legierung mit mindestens einem dieser Metalle sein. Diese Materialien gehören zur Gruppe biokompatibler Materialien. Beispielsweise kann eine TiCuNi-Legierung eingesetzt werden.

Gemäß einer günstigen Ausgestaltung kann der Flansch metallisch leitend sein, und vorzugsweise aus einem Metall bestehen, das weitgehend dem Metall eines Gehäuses eines Therapiegerätes entspricht, für das die Durchführung vorgesehen ist. mit einem metallisch leitenden Flansch lässt sich durch Hartlöten, Weichlöten oder Schweißen eine hermetisch dichte Verbindung mit dem Substrat herstellen. Ist der Flansch aus dem Gehäusematerial gebildet, ist eine stoffschlüssige und damit hermetisch dichte Verbindung zwischen Durchführung und Gehäuse besonders einfach und zuverlässig darstellbar.

Gemäß einer günstigen Ausgestaltung kann das Substrat ein elektrisches Filterbauelement umfassen. Dies ermöglicht eine verbesserte Entstörung von Anbindungen an beispielsweise Antennen, wobei die Signale vollständig auf einer geschlossenen Platine geführt werden können. Es können aktive oder auch passive Filterschaltungen unmittelbar in der Nähe der Durchführung platziert werden. Vorteilhaft ist ein Filterbauelement z.B. für so genannte Home Monitoring Systeme, bei denen das Implantat Daten vom Implantationsort beispielsweise an einen Arzt überträgt.

Gemäß einem weiteren Aspekt der Erfindung wird ein elektrisches Kontaktelement für ein elektromedizinisches Implantat mit einer erfindungsgemäßen elektrischen Durchführung vorgeschlagen, mit einem Substrat und wenigstens einem Flansch, der hermetisch dicht mit dem Substrat verbunden ist. Vorteilhaft ist die elektrische Verbindung gesteckt und kann schnell geöffnet oder geschlossen werden. Für eine dauerhafte Verbindung kann eine gesteckte Verbindung definiert fixiert werden, z. B. durch Schweißen, Löten, insbesondere Hartlöten, Weichlöten, Verformen oder Ähnliches.

Dabei kann das Substrat eine Platine, insbesondere eine bedruckte PCB-Platine sein. Diese kann so geformt sein, dass ein schmaler Ausläufer als erster Bereich des Substrats durch den Flansch durchgeführt ist und einen großflächigeren Bereich als zweiter Bereich Bauelemente und/oder Schaltungen aufweist, die Funktionen des elektromedizinischen Implantats erfüllen. Von Vorteil ist es, wenn Platinen eingesetzt werden, welche ausschließlich biokompatible Werkstoffe enthalten, z.B. mit Al₂O₃ als Trägermaterial und Niob für Leiterbahnen.

Gemäß einer günstigen Ausgestaltung kann die Durchführung an eine separate Platine angekoppelt sein. Damit können das Substrat und die Durchführung relativ einfach aufgebaut sein. Komplexere Funktionen können auf der separaten Platine integriert sein. Hierdurch können Kontaktelement-Gleichteile für unterschiedlichste Platinen bzw. Implantate kostensparend in großer Stückzahl hergestellt werden.

Gemäß einem weiteren Aspekt der Erfindung wird ein elektromedizinisches Implantat, insbesondere ein implantierbares Elektrotherapiegerät, vorgeschlagen, insbesondere ein Herzschrittmacher oder Kardioverter/Defibrillator, mit einem erfindungsgemäßen elektrischen Kontaktelement.

Vorteilhaft sind daneben auch elektromedizinische Implantate wie beispielsweise Defibrillatoren, Neurostimulatoren, Herzschrittmacher und Cochlea-Implantate mit einem oder mehreren elektrischen Kontaktelementen nach der Erfindung.

Die Erfindung ist nachfolgend beispielhaft, anhand von in Zeichnungen dargestellten Ausführungsbeispielen, näher erläutert. Es zeigen in schematischer Darstellung:
- Fig. 1: als Draufsicht einen Querschnitt durch ein Ausführungsbeispiel eines elektromedizinischen Implantats mit einem elektrischen Kontaktelement mit einer Durchführung mit Leiterbahnen auf einem Substrat als elektrischen Kontaktelementen;
- Fig. 2: einen Blick auf eine Stirnseite des Ausführungsbeispiels aus Figur 1;
- Fig. 3: als Explosionszeichnung eine Durchführung vor dem Einsetzen in einen Flansch;
- Fig. 4: Varianten von möglichen Kontaktelementen und Schaltungen auf und in einem Substrat einer Durchführung vereinigt in einer einzigen Durchführung mit Stufen und Ausnehmungen zum verwechslungssicheren Einbau;
- Fig. 5: einen Schnitt durch ein Substrat mit innerhalb des Substrats geschirmten Leiterbahnen;
- Fig. 6: einen Schnitt durch ein Substrat mit innerhalb des Substrats geschirmten Leiterbahnen und Durchkontaktierungen zwischen verschiedenen Lagen des Substrats;
- Fig.7: als Draufsicht ein geformtes Substrat mit Flansch und Schirmung von elektrischen Kontaktelementen in Form von Leiterbahnen auf einem ersten Bereich des Substrats;
- Fig. 8: eine Variante eines ersten Bereichs einer Durchführung;
- Fig. 9: eine weitere Variante eines ersten Bereichs einer Durchführung zum Befestigen an eine Platine;
- Fig. 10: eine weitere Variante eines ersten Bereichs einer Durchführung zum Befestigen an eine Platine;
- Fig. 11: einen Schnitt durch einen Flansch mit durchgeführtem Substrat, das mittels eines metallischen Lots hermetisch dicht mit dem Flansch verbunden ist mit einer Leiterbahn an der Oberfläche des Substrats und Abdeckschicht über der Leiterbahn;
- Fig. 12: einen Schnitt durch einen Flansch mit durchgeführtem Substrat, das hermetisch dicht mittels eines metallischen Lots mit dem Flansch verbunden ist mit einer Masseleitung an der Oberfläche des Substrats;
- Fig. 13: einen Schnitt durch einen Flansch mit durchgeführtem Substrat, das hermetisch dicht mittels eines Glaslots mit dem Flansch verbunden ist;
- Fig. 14: eine doppelte Verbindung eines Substrats mit einem Flansch;
- Fig. 15: eine Draufsicht auf Signalleitungen auf einem Substrat, welche mit einer Masseleitung umgeben sind;
- Fig. 16: ein vereinfachtes Ersatzschalbild der Anordnung in Figur 15; und
- Fig. 17: einen Schnitt durch ein mehrlagiges Substrat mit mehreren Ebenen von Masseleitungen.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typische Ausgestaltungen der Erfindung wieder und sollen die Erfindung nicht auf die dargestellten Ausgestaltungen beschränken.

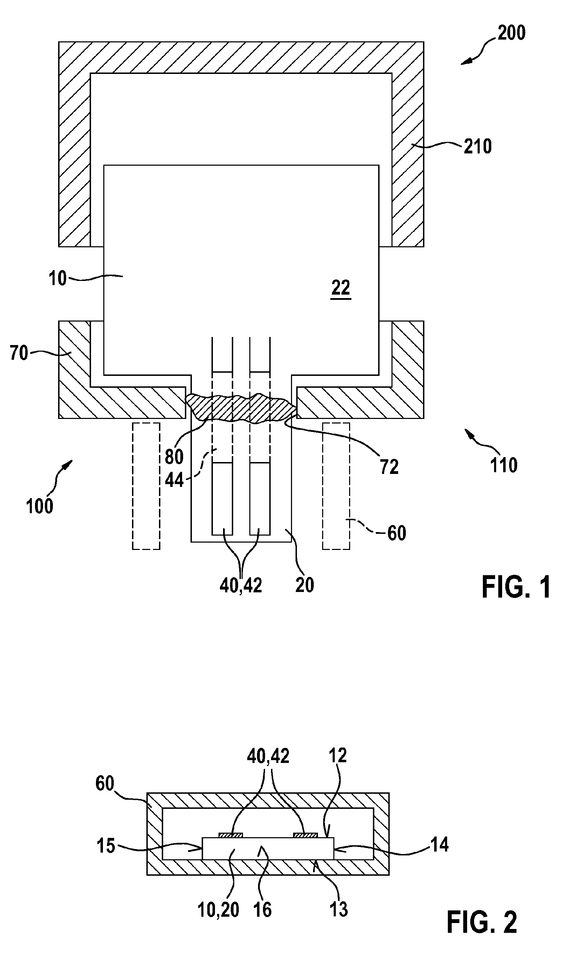

Figur 1 zeigt als Draufsicht einen Querschnitt durch ein Ausführungsbeispiel eines elektromedizinischen Implantats 200 mit einem elektrischen Kontaktelement 110 mit einer Durchführung 100 mit zwei Leiterbahnen 42 auf einem Substrat 10 als elektrischen Kontaktelementen 40.

Die Leiterbahnen 42 erstrecken sich zumindest bereichsweise in oder auf einem elektrisch isolierenden Substrat 10 von einem ersten Bereich 20 zu einem zweiten Bereich 22. Der erste Bereich 20 bildet einen Außenkontakt, an den ein Gegenkontakt angeschlossen werden kann (nicht dargestellt). Beispielsweise stellt der erste Bereich 20 des Substrats 10 den Kontakt eines Steckers dar, bei dem die Leiterbahnen 42 als elektrische Kontaktelemente dienen. Die Leiterbahnen 42 sind beispielsweise aus einer Platin-Iridium-Legierung gebildet oder einem anderen biokompatiblen Material. Die Leiterbahnen 42 sind fest mit dem Substrat 10 verbunden und stehen nicht frei über das Substrat 10 hinaus, abgesehen von ihren Dicken senkrecht zum Substrat, so dass auf diese Weise die absolute und relative Lage der Leiterbahnen 42 zueinander fest vorgegeben ist.

Das Substrat 10 ist beim Übergang vom ersten Bereich 20 in den zweiten Bereich 22 in diesem Ausführungsbeispiel durch einen Flansch 70 geführt. Der Flansch 70 ist insbesondere ein Metallflansch, vorzugsweise aus Titan oder einem anderen biokompatiblen metallischen Werkstoff, welcher vorzugsweise in seiner Zusammensetzung mit dem Material des Implantatgehäuses 210 weitgehend übereinstimmt.

Das Substrat 10 ist mit dem Flansch 70 hermetisch dicht verbunden, indem ein Lot 80 in den Zwischenraum zwischen Substrat 10 und Flanschöffnung 72 eingebracht ist. Das Substrat 10 kann mit dem Flansch beispielsweise gefügt oder verlötet sein.

Im Bereich des Durchtritts durch den Flansch 70 verlaufen die Leiterbahnen 42 unterhalb der Substratoberfläche als vergrabene Leiterbahnen 44, so dass kein elektrischer Kontakt zwischen Flansch 70 und Leiterbahnen 42 besteht, wenn ein metallisches Lot 80 eingesetzt wird. Alternativ können die Leiterbahnen 42 mit einer Isolationsschicht abgedeckt sein und/oder es kann ein elektrisch isolierendes Glaslot verwendet werden, wobei in diesem Fall die Leiterbahnen 42 nicht als vergrabene Ausführung 44 ausgebildet sein müssen.

Der erste Bereich 20 des Substrats 10 ist im gezeigten Beispiel geschirmt, indem Oberseite 12 (in der Figur 1 in Draufsicht zu sehen) und Unterseite 13 und die beiden Seitenflächen 14, 15 des Substrats 10 von einer metallischen Schirmung 60 umgeben sind. Figur 2 zeigt einen Blick auf die Stirnseite 16 des Substrats 10 im ersten Bereich 20. Die Schirmung 60 schützt einerseits den ersten Bereich 20 des Substrats 10 und dient andererseits zur optionalen festen Verbindung mit einem Gegenstecker.

Möglichkeiten zur Herstellung des Substrats 10 sind, eine Platine aus Al₂O₃, wie sie aus der Leistungselektronik bekannt ist, Kontur zu fräsen oder bereits dem Grünling diese Kontur zu geben. Statt einer solchen HTCC-Keramik kann alternativ eine LTCC-Keramik vorgesehen sein, bei der in diesem Fall der Grünling aus einer Mischung aus Keramikpulver, Glasfritte und organischem Binder zusammengesetzt ist.

Eine HTCC-Keramik ist üblicherweise dann geeignet, wenn für die Leiterbahnen 42 höher schmelzende Materialien wie Mo, W, Ta, Nb oder Legierungen daraus als Metalle eingesetzt werden. Übliche Sintertemperaturen liegen in diesem Fall im Bereich von 1400°C bis 1500°C. LTCC-Keramiken kommen dann in Betracht, wenn ein relativ niedrig schmelzendes Metall für die Leiterbahnen 42 oder Kontakte 40 etc., z.B. Au, Pt, Ir, Pd oder Legierungen daraus eingesetzt wird, so dass die Sintertemperaturen unterhalb der Schmelztemperatur des Metalls liegen müssen. Typische Prozesstemperaturen liegen dann um 900°C. Als Flanschmaterial ist Ti bevorzugt. Das Lot 80, mit dem der Flansch 70 hermetisch dicht mit dem Substrat 10 verbunden wird, weist einen geringeren Schmelzpunkt bzw. Schmelzbereich als der Flansch 70 auf, wenn es sich um ein metallisches Lot handelt. Wenn das Lot 80 aus Glas besteht, dann liegt die Verarbeitungstemperatur des Glases unterhalb der Schmelztemperatur des Flansches 70.

Werden im Außenbereich 20 nicht biokompatible Materialien für die Leiterbahnen 42 bzw. die Kontakte 40 verwendet, werden die Leiterbahnen 42 bzw. die Kontakte 40 mit einem biokompatiblen Metall bzw. einer biokompatiblen Metalllegierung so beschichtet, so dass die nicht biokompatiblen Bereiche der Leiterbahnen 42 bzw. der Kontakte 40 vollständig und lückenlos von biokompatiblem Material wie z. B. Au, Pt, Pd, Ir, Ti, Nb, Ta oder Legierungen daraus umschlossen sind. Mögliche Verfahren zur Aufbringung einer solchen Beschichtung sind von der Wahl des Grundmaterials der Leiterbahnen 42 abhängig und sind z. B. galvanisieren oder mittels Maskenprozess im PVD-Verfahren, d. h. mittels Aufdampfen oder Kathodenzerstäuben. Alternativ kann auf Platinenmaterial zurückgegriffen werden, dessen Leiterbahnen 42 bereits aus biokompatiblem Material bestehen. Die entstehende Platine kann optional noch im Reflow- und SMT Verfahren mit elektrischen Bauelementen bestückt und verlötet werden. Abschließend wird die Platine in einen biokompatiblen Flansch 70 eingesetzt. Der Flansch kann durch Drehen, Fräsen, MiM-Technik (Metall-Isolator-Metall-Technik), Sintern oder andere Verfahren hergestellt sein. Mit einem entsprechendem Lot 80 (Glaslot oder z. B. Goldlot) wird die Platine 10 in den Flansch 70 eingeschmolzen bzw. gefügt. Dabei ist zweckmäßigerweise die Maßhaltigkeit sowie die Spannungsverteilung der Materialpaarung zu beachten, um dauerhaft hermetisch dichte Durchführungen 100 zu bilden. Abschließend kann die Durchführung 100 mit dem restlichen Gehäuse 210 hermetisch dicht und mechanisch fest verbunden werden, beispielsweise mittels Hartlöten, Weichlöten, Schweißen, insbesondere Laserschweißen.

So kann z.B. eine Durchführung 100 mit der Elektronik eines so genannten Leadless Herzschrittmachers hergestellt werden und direkt ins Gehäuse 210 gefügt werden, wodurch Kosten gespart und flexible, steckbare Elektroden eingespart werden können.

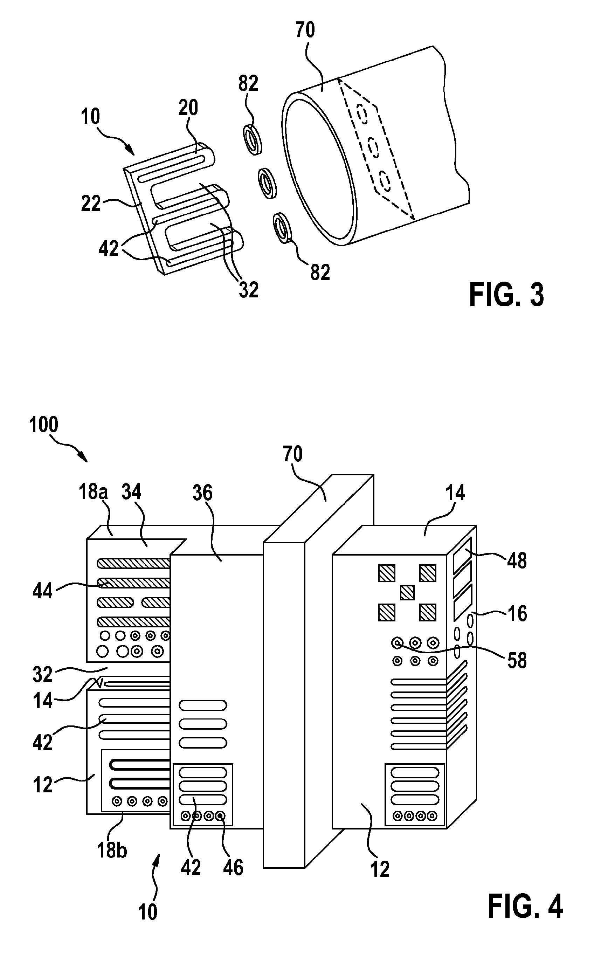

Figur 3 zeigt als Explosionszeichnung eine Variante einer Durchführung 100 mit dem Substrat 10 vor dem Einsetzen in einen Flansch 70, entsprechend einer Durchsteckvariante. Der Flansch 70 kann in eine Öffnung eines Implantats hermetisch dicht und mechanisch fest angefügt werden.

In diesem Beispiel ist der erste Bereich 20 des Substrats 10 so ausgebildet, dass zwischen den Leiterbahnen 42 Ausnehmungen 32 im Substrat 10 vorgesehen sind. Die Ausnehmungen 32 können aus dem Grünling gestanzt oder gefräst sein. Der erste Bereich 20 erhält dadurch eine kammartige Struktur. Der zweite Bereich 22 kann eine gerade Kante ohne Ausnehmungen aufweisen. Über die einzelnen Kammzinken werden Lotringe 82 gesteckt und mittels eines Hochtemperaturlötprozesses mit dem Flansch 70 hermetisch dicht und mechanisch fest verbunden. Die Leiterbahnen 42 im ersten Bereich 20 bilden elektrische Kontakte 40 für eine elektrische Verbindung mit einem entsprechenden Gegenelement.

Das Substrat 10 kann ein passiver Träger für die Leiterbahnen 42 sein oder einen oder mehrere Schaltkreise enthalten. Die Leiterbahnen 42 können - wie bei Figur 1 beschrieben - gefertigt sein und im Falle nicht biokompatibler Leiterbahnen biokompatible Beschichtungen aufweisen, die galvanisch oder mittels üblicher PVD- oder CVD-Schichtabscheideverfahren wie Aufdampfen, Kathodenzerstäubung bzw. plasmaunterstützte Verfahren abgeschieden werden.

Figur 4 zeigt Varianten von möglichen Kontakten 40, Leiterbahnen 42 und Schaltungen auf und in einem Substrat 10 einer Durchführung 100 beispielhaft vereinigt in einer einzigen Durchführung 100. Das Substrat 10 ist in einem Bereich mit Stufen 34, 36 versehen, so dass das Substrat 10 am freien Ende dünner ist als am Flansch 70. Ferner ist dort eine Ausnehmung 32 angeordnet, welche das Substrat 10 in zwei Segmente 18a, 18b teilt. Beides, Stufen 34, 36 und Ausnehmung 32, können jeweils zum verwechslungssicheren Einbau des Substrats 10 bzw. der Durchführung 100 genutzt werden.

Es können Leiterbahnen 42 an der Oberseite 12 (und/oder der Unterseite) des Substrats 10 vorgesehen sein oder auch an Seitenwänden 14. Es können vergrabene Leiterbahnen 44 innerhalb des Substrats 10 verlaufen. Es können Masseleitungen 46 um Leiterbahnen 42 angeordnet sein. Ferner können Durchkontaktierungen 58 (Vias) vorgesehen sein, d.h. Öffnungen, in die Lot eingebracht werden kann, um Leiterbahnen auf zwei verschiedenen Substratlagen miteinander zu verbinden. Weiterhin können Öffnungen zu Hohlräumen im Substrat führen, die mit Metalllot verfüllt werden können, womit sich elektrische und biokompatible Eigenschaften der Leiterbahnen und Kontakte des Substrats 10 gezielt einstellen lassen.

Leiterbahnen können über eine Kante geführt werden, wie auf der rechten Bildhälfte dargestellt ist. Ebenso können Kontaktpads 48 an der Stirnseite 16 des Substrats angeordnet sein.

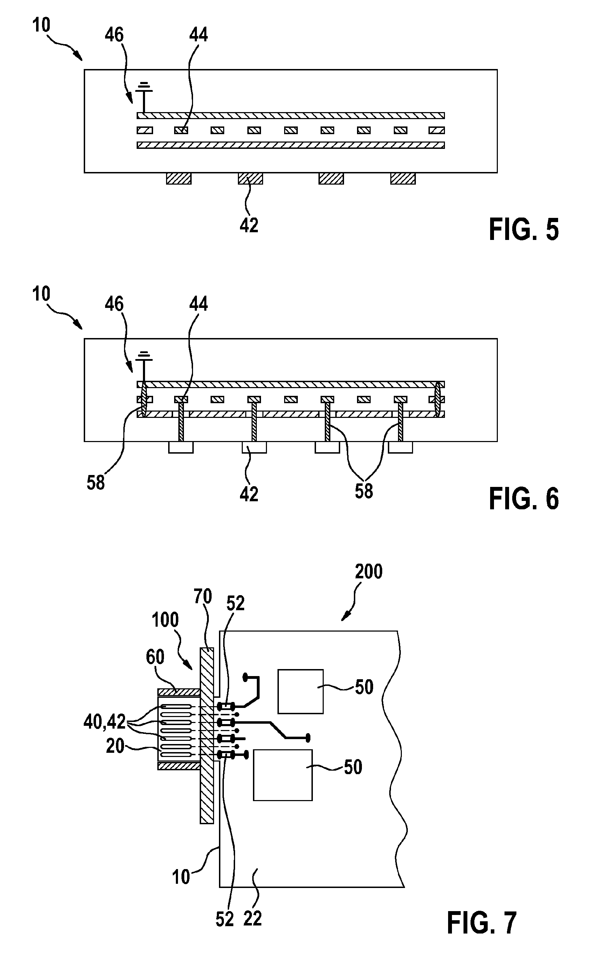

Die Figuren 5 und 6 illustrieren in Schnittdarstellungen Möglichkeiten, wie eine Schirmung im Inneren des Substrats 10 bewirkt werden kann.

Figur 5 zeigt einen Schnitt durch das Substrat 10, bei dem Leiterbahnen 44 zwischen zwei flächigen Potenzialleitungen 46 verlaufen, wobei die beiden äußeren Leiterbahnen auf den Potenzialleitungen zugeordnet sind. Die flächigen Potenzialleitungen 46 können auch als Gitter ausgeführt sein (nicht dargestellt), so dass einerseits mechanische Spannungen in dem Aufbau gemindert werden und andererseits hiermit die Funktion eines sog. "Faradayschen Käfigs" weiterhin gewährleistet bleibt. Eine der Potenzialleitungen 46 liegt z.B. auf Masse. Die andere Potenzialleitung kann auf Masse gelegt sein oder auf ein anderes Potenzial. An eine Oberfläche des Substrats 10 sind über der geschirmten Anordnung Leiterbahnen 42 geführt. Figur 6 zeigt eine geschirmte Anordnung von Leiterbahnen 44 innerhalb des Substrats 10, bei denen Durchkontaktierungen 58 zwischen den beiden flächigen Potenzialleitungen 46 diese sowie die beiden seitlichen Potenzialleitungen miteinander verbinden, so dass die Anordnung aus vergrabenen Leiterbahnen 44 von einem Massekäfig umschlossen ist. Die Leiterbahnen 42 an der Oberfläche des Substrats 10 sind über Durchkontaktierungen 58 mit den Leiterbahnen 44 im Innern des Massekäfigs verbunden. Hierzu sind entsprechende Öffnungen in einer der flächigen Masseleitungen 46 vorgesehen.

Figur 7 zeigt als Draufsicht schematisch ein elektromedizinisches Implantat 200 (ohne Gehäuse dargestellt) mit einem Außenkontur geformten Substrat 10, das einen ersten Bereich 20 auf einer Seite eines Flansches 70 aufweist und einen zweiten Bereich 22 auf der anderen Seite des Flansches 70. Der erste Bereich 20 steht als Zunge vom zweiten Bereich 22 ab und ist mit einer am freien Ende offenen Schirmung 60 umgeben. Mehrere Leiterbahnen 42 bilden elektrische Kontakte 40 für eine Gegenkontakteinrichtung, etwa einen Stecker bzw. eine Buchse. Die Leiterbahnen 42 sind vom ersten Bereich 20 zum zweiten Bereich 22 elektrisch isoliert durch den Flansch 70 geführt, indem diese z.B. mit einer nicht näher bezeichneten Isolierschicht abgedeckt sind bzw. im Bereich des Flansches in einer inneren Lage des Substrates 10 vergraben sind. Die Kontakte 42 können in ihrem Auslauf dicker ausgeführt sein als die Leiterbahnen 42, so dass beim Stecken mit einem Gegenstecker die Kontakte nicht abgerieben werden, sondern sichere elektrische Kontaktierungen gewährleisten. Der zweite Bereich 22 ist als flächige Platine ausgebildet und kann z. B. eine mikrostrukturierte Schaltungsanordnung aufweisen. Auf dieser sind an der Oberfläche SMD-Bauelemente montiert. Beispielsweise sind die Leiterbahnen 42 flanschnah mit Kondensatoren 52 an die Platine angebunden, um eine effiziente Schirmung vor elektromagnetischer Störstrahlung zu realisieren. Zwei Mikrochips 50 sind beispielhaft vorgesehen.

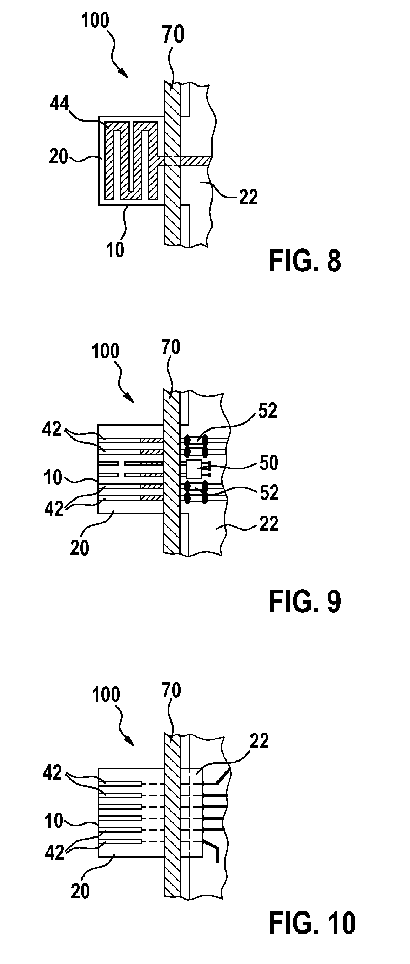

Die Figuren 8 bis 10 zeigen verschiedene alternative Durchführungen 100 für ein elektromedizinisches Implantat 200 aus Figur 7. Figur 8 zeigt eine vergrabene Antenne in Form einer mäanderförmigen Leiterbahn 44 im ersten Bereich 20 des Substrats 10. Das Substrat 10 ist wie in Figur 7 konturgeformt, und die Antenne ist innerhalb des Substrats 10 zum zweiten Bereich 22 geführt. Die Antenne kann je nach elektrischer Anforderung als Stab- oder als Loopantenne ausgeführt sein.

In der Variante in Figur 9 ist die Durchführung 100 zur Befestigung an einer separaten Platine (nicht dargestellt) vorgesehen. Das Substrat 10 ist rechteckförmig und weist flanschnah an seinem zweiten Bereich 22 SMD-Bauelemente auf in Form von Kondensatoren 52 und einem IC-Bauelement 50. Je zwei äußeren Leiterbahnen 42 mit den flanschnahen Kondensatoren sind innerhalb der Platine 10 durch den Flansch 70 geführt, wobei von den Kondensatoren 52 weg Anschlüsse gebildet sind, an denen Bonddrähte angeordnet sind, mit denen eine elektrische Verbindung zu der nicht dargestellten Platine herstellbar sind. Mittels der Kondensatoren 52 kann z. B. ein Tiefpass realisiert werden. Zwischen den äußeren Leiterbahnen 42 ist eine zweigeteilte Leiterbahnstruktur angeordnet, der im zweiten Bereich das IC-Bauelement 50 zugeordnet ist, von dem eine Mehrzahl an Leiterbahnen zu Anschlüssen weggeführt. Die Anschlüsse des zweiten Bereichs 22 dienen zur Verbindung mit der nicht dargestellten separaten Platine.

In der Variante in Figur 10 zeigt wie in Figur 9 eine Durchführung 100, die zur Befestigung an einer separaten Platine (nicht dargestellt) vorgesehen ist. Im ersten Bereich 20 des Substrats 10 sind eine Anzahl von Leiterbahnen 42 als elektrische Kontakte 40 vorgesehen, die nicht wie in den Figuren 7 bis 9 äquidistant, sondern in Querrichtung mit abnehmendem Abstand auf dem Substrat 10 angeordnet sind. Im zweiten Bereich 22 sind stirnseitig am Substrat 10 Anschlüsse vorgesehen, an denen Bonddrähte zur Kontaktierung einer nicht dargestellten Platine vorhanden sind. Die Verbindung der Leiterbahnen 42 im ersten Bereich zu den stirnseitigen Anschlüssen verläuft innerhalb des Substrats 10 so, dass kein elektrischer Kurzschluss zwischen dem Flansch 70 und den Leiterbahnen 42 entstehen kann.

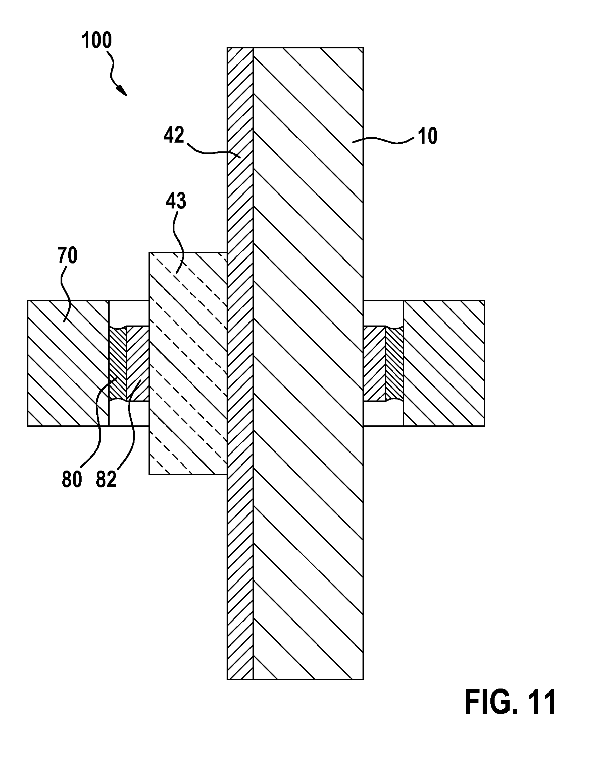

Figur 11 zeigt einen Schnitt durch eine Durchführung 100 mit einem Flansch 70 mit durchgeführtem Substrat 10 in der Art der Ausführungsbeispiele der Figuren 7 bis 10. Der Flansch 70 ist mittels eines metallischen Lots 80, z. B. Goldlot oder einer TiCuNi-Legierung, hermetisch dicht mit dem Substrat 10 verbunden. An der Oberseite des Substrats 10 ist beispielhaft eine Leiterbahn 42 geführt. Im Bereich des Flansches 70 ist die Leiterbahn 42 mit einer elektrisch isolierenden Abdeckschicht 43 abgedeckt bzw. das Substrat 10 stufig ausgeführt, so dass die Leiterbahn 42 in diesem Bereich vergraben ist.

Zur besseren Benetzung des Substrats 10 und der Abdeckschicht 43 durch das Lot 80, wie Gold, ist eine geeignete Haftvermittlerschicht 82 unter dem Lot 80 auf dem Substrat 10 und auf der Abdeckschicht 43 vorgesehen. Im Falle der elektrisch leitenden Metalllegierung TiCuNi als Lot 80 kann die Haftvermittlerschicht 82 entfallen, da dieses Lot in der Lage ist, das keramische Substrat 10 direkt zu benetzen. Das Material für die Haftvermittlerschicht kann je nach vorhandener Materialpaarung geeignet gewählt werden und besteht z. B. aus Nb, Ti, Ti/Mo, Ta, etc.

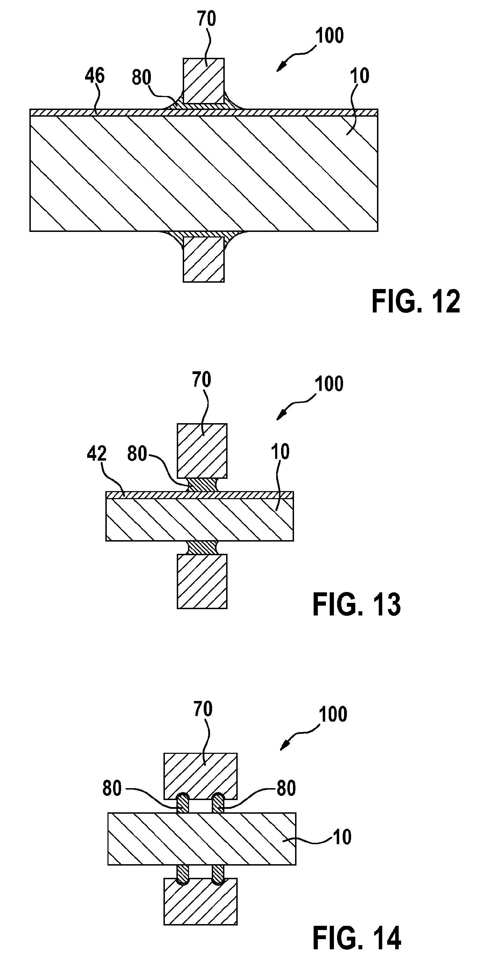

Figur 12 zeigt einen Schnitt durch eine Durchführung 100 mit einem Flansch 70 mit durch den Flansch70 geführtem Substrat 10 in der Art der Ausführungsbeispiele der Figuren 7 bis 10. Der Flansch 70 ist mit dem Substrat 10 mittels eines metallischen Lots 80 hermetisch dicht verbunden. An der Oberfläche des Substrats 10 ist eine Masseleitung 46 angeordnet, so dass das Lot 80 direkt mit der Masseleitung 46 und dem Substrat 10 verbunden sein kann.

Figur 13 zeigt eine alternative Ausgestaltung zu Figur 12, bei der statt eines metallischen Lots 80 ein Glaslot verwendet ist, um das Substrat 10 hermetisch dicht mit dem Flansch 70 zu verbinden. Die Leiterbahn 42 an der Substratoberfläche kann daher direkt vom Glaslot kontaktiert werden, ohne einen elektrischen Kurzschluss zum Flansch 70 herzustellen.

Figur 14 zeigt eine Variante einer Durchführung, bei der der Flansch 70 mit zwei in axialer Richtung des Flanschs 70 hintereinander angeordneten Dichtungen, welche hermetisch dicht mit dem Substrat 10 verbunden sind. Die Dichtungen sind jeweils durch eine Lotverbindung mit einem Lot 80 erzeugt. Mit dieser Variante ist eine erhöhte Sicherheit der hermetischen Verbindung zwischen Flansch 70 und Substrat 10 erreichbar: Wenn eine der beiden hermetisch dichten Verbindungen z. B. durch einen Riss undicht wird, dann ist der Riss auf die eine Dichtung beschränkt, weil er sich nicht zur zweiten fortsetzen kann, und die zweite Dichtung ist intakt, so dass die Hermetizität sicherer gewährleistet bleibt als mit einer einfachen Dichtung.

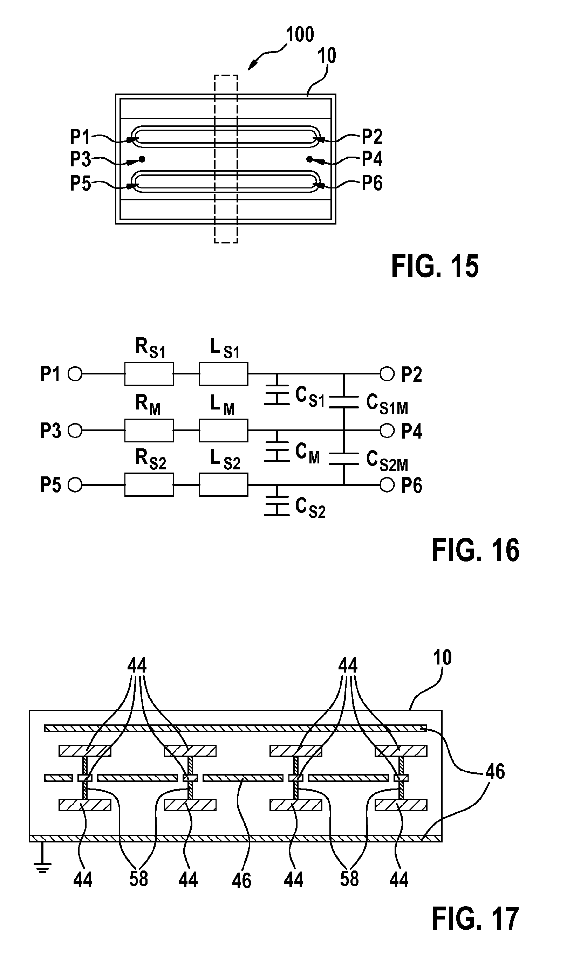

Die Figuren 15 und 16 zeigen als Draufsicht (Figur 15) und als vereinfachtes Ersatzschaltbild (Figur 16) zwei Signalleitungen mit Endpunkten P1, P2 und P5, P6, die auf einem Substrat 10 angeordnet und von einer Masseleitung mit Kontaktpunkten P3, P4 umgeben sind.

Die Signalleitung zwischen den Endpunkten P1, P2 setzt sich im Ersatzschaltbild aus einem Widerstand R_{S1}, einer Induktivität L_{S1}, einer Kapazität C_{S1}, die abhängig vom Substrat 10 ist, sowie einer Kapazität C_{S1M} zusammen. Die Signalleitung zwischen den Endpunkten P5, P6 setzt sich im Ersatzschaltbild aus einem Widerstand R_{S2}, einer Induktivität L_{S2}, einer Kapazität C_{S2}, die abhängig vom Substrat 10 ist, sowie einer Kapazität C_{S2M} zusammen. Die Masseleitung mit den Kontaktpunkten P3, P4 setzt sich im Ersatzschaltbild aus einem Widerstand R_{M}, einer Induktivität L_{M}, einer Kapazität C_{M} zusammen.

Zum Einstellen günstiger und geplanter Parameter können grundsätzlich alle bekannte Verfahren und Technologien verwendet werden, wie etwa Ätzen, Beschichten, Drucken, Durchkontaktieren und dergleichen.

Figur 17 zeigt den Schnitt durch ein mehrlagiges Substrat 10 mit mehreren Masse-Ebenen 46 mit Masseleitungen oder Masseflächen. Die Fläche der Leiterbahnen 44 im Innern des Substrats 10 wurde durch zusätzliche Lagen und Durchkontaktierungen 58 vergrößert. Beispielsweise sind drei Lagen mit Leiterbahnen 44 vorgesehen. Durch deren Ausrichtung und Position zu den Masseebenen 46 können die elektrischen Eigenschaften, wie z.B. Dämpfung, Schirmung, gezielt variiert werden. Die jeweiligen Flächen von Masse 46 und Leiterbahnen 44 können als Vollflächen, Mäander oder Beschichtung aufgebracht werden.

### Bezugszeichenliste

- 10: Substrat
- 12,13: Oberfläche
- 14,15: Oberfläche
- 16: Oberfläche
- 18a,b: Segment
- 20: Bereich
- 22: Bereich

- 30: Platine
- 32: Ausnehmung
- 34: Stufe
- 36: Stufe

- 40: Elektrischer Kontakt
- 42: Leiterbahn
- 43: Isolierschicht
- 44: Verborgene Leiterbahn
- 46: Masse
- 48: Kontaktfeld
- 50: Mikrochip
- 52: Kondensator
- 54: Schirmung
- 56: Filter
- 58: Durchkontaktierung
- 60: Schirmung
- 62: Gedruckte Schaltung

- 70: Flansch
- 72: Öffnung

- 80: Lot
- 82: Schicht
- 100: Durchführung

- 110: Kontaktelement

- 200: Elektrisches Implantat
- 210: Implantatgehäuse

## Patentansprüche

1. Elektrische Durchführung (100) für elektromedizinische Implantate, umfassend mindestens einen elektrischen Kontakt (40) zum Verbinden mit einem Gegenkontakt, **dadurch gekennzeichnet, dass** der mindestens eine elektrische Kontakt (40) als Leiterbahn (42) ausgebildet ist, die sich zumindest bereichsweise in oder auf einem elektrisch isolierendem Substrat (10) von einem ersten Bereich (20) zu einem zweiten Bereich (22) erstreckt, wobei das Substrat (10) beim Übergang vom ersten in den zweiten Bereich (20, 22) durch einen Flansch (70) geführt ist, und dass das Substrat (10) mit dem Flansch (70) hermetisch dicht verbunden ist.

2. Durchführung nach Anspruch 1, **dadurch gekennzeichnet, dass** zur hermetisch dichten Verbindung ein Lot (80), insbesondere ein Glaslot oder Metalllot, vorgesehen ist.

3. Durchführung nach Anspruch 1 oder 2, **dadurch gekennzeichnet, dass** das Substrat (10) eine Leiterplatte, insbesondere eine keramische Leiterplatte, ist.

4. Durchführung nach einem der vorhergehenden Ansprüche, **dadurch gekennzeichnet, dass** das Substrat (10) ein oder mehrere gedruckte Schaltungen (62) und/oder ein oder mehrere SMD-Bauelemente (50) aufweist.

5. Durchführung nach einem der vorhergehenden Ansprüche, **dadurch gekennzeichnet, dass** wenigstens der erste Bereich (20) des Substrats (10) eine elektromagnetische Schirmung aufweist.

6. Durchführung nach einem der vorhergehenden Ansprüche, **dadurch gekennzeichnet, dass** das Substrat (10) ein oder mehrere elektrische Kontakte (40) auf einer oder mehreren Oberflächen (12, 13, 14, 15, 16) aufweist.

7. Durchführung nach einem der vorhergehenden Ansprüche, **dadurch gekennzeichnet, dass** das Substrat (10) im ersten und/oder zweiten Bereich (20, 22) eine gestufte Oberfläche aufweist mit wenigstens zwei Stufen (34, 36).

8. Durchführung nach einem der vorhergehenden Ansprüche, **dadurch gekennzeichnet, dass** das Substrat (10) im ersten und/oder zweiten Bereich (20, 22) wenigstens eine Ausnehmung (32) so aufweist, dass zwei oder mehr voneinander beabstandete Substratsegmente (18a, 18b) gebildet sind.

9. Durchführung nach einem der vorhergehenden Ansprüche, **dadurch gekennzeichnet, dass** das Substrat (10) und/oder die elektrischen Kontakte (40) zumindest bereichsweise aus biokompatiblem Material gebildet ist oder zumindest bereichsweise durch biokompatibles Material gekapselt ist.

10. Durchführung nach einem der vorhergehenden Ansprüche, **dadurch gekennzeichnet, dass** eine Zuordnung der elektrischen Kontakte (40) in den beiden Bereichen (20, 22) unterschiedlich bzw. vertauscht ausgeführt ist, indem die Leiterbahnen (42) im Substrat (10) in verschiedenen Leiterbahn-Ebenen mittels Durchkontaktierungen elektrisch isoliert und gekreuzt ausgeführt werden.

11. Durchführung nach einem der vorhergehenden Ansprüche, **dadurch gekennzeichnet, dass** der Flansch (70) metallisch leitend ist, und vorzugsweise aus einem Metall besteht, das weitgehend dem Metall eines Gehäuses (110) eines Therapiegerätes entspricht, für das die Durchführung (100) vorgesehen ist

12. Durchführung nach einem der vorhergehenden Ansprüche, **dadurch gekennzeichnet, dass** das Substrat (10) mindestens ein elektrisches Filterbauelement (56) umfasst.

13. Elektrisches Kontaktelement (110) für ein elektromedizinisches Implantat (200) mit einer elektrischen Durchführung (100) nach einem der vorhergehenden Ansprüche mit einem Substrat (10) und wenigstens einem Flansch (70), der hermetisch dicht mit dem Substrat (10) verbunden ist.

14. Kontaktelement nach Anspruch 13, **dadurch gekennzeichnet, dass** die Durchführung (100) an eine Platine (30) angekoppelt ist.

15. Elektromedizinisches Implantat (200), insbesondere Herzschrittmacher oder Kardioverter/Defibrillator, mit einem elektrischen Kontaktelement (110) nach einem der Ansprüche 12 bis 13.
